Europäisches Patentamt

European Patent Office

Office européen des brevets

(19)

(11) Numéro de publication : **0 395 684 B1**

(12) **FASCICULE DE BREVET EUROPEEN**

(45) Date de publication du fascicule du brevet :
02.09.92 Bulletin 92/36

(51) Int. Cl.⁵ : **A61M 5/00**, B65F 1/16

(21) Numéro de dépôt : **88910049.1**

(22) Date de dépôt : **07.11.88**

(86) Numéro de dépôt international :
**PCT/FR88/00543**

(87) Numéro de publication internationale :
**WO 89/04182 18.05.89 Gazette 89/11**

(54) **BOITE RECUPERATRICE DE DECHETS DANGEREUX.**

(30) Priorité : **06.11.87 FR 8715803**

(43) Date de publication de la demande :
**07.11.90 Bulletin 90/45**

(45) Mention de la délivrance du brevet :
**02.09.92 Bulletin 92/36**

(84) Etats contractants désignés :
**AT BE CH DE FR GB IT LI LU NL SE**

(56) Documents cités :
**EP-A- 0 080 882
CH-A- 418 222
DE-A- 3 505 892
US-A- 2 917 210
US-A- 3 240 373
US-A- 4 375 849
US-A-37 928 03**

(73) Titulaire : **CENTRE SPECIALITES
PHARMACEUTIQUES C.S.P. (S.A.)
76 avenue du Midi Z.I.
F-63800 Cournon (FR)**

(72) Inventeur : **BAUDRY, Jean-Paul
100, avenue Joseph-Claussat
F-63400 Chamalières (FR)**
Inventeur : **BOURG, Joelle
18 bis, boulevard Barrieu
F-63130 Royat (FR)**

(74) Mandataire : **Chanet, Jacques
Conseil en Brevets 129 Avenue de Royat B.P.
27
F-63400 Chamalières (FR)**

Jouve, 18, rue Saint-Denis, 75001 PARIS

# Description

La présente invention est du domaine des conteneurs et elle a plus précisément pour objet une boîte de récupération de déchets et de petits objets jetables et dangereux tels que notamment petits instruments et déchets des hôpitaux.

On connaît des conteneurs de récupération de déchets divers tels par exemple que verre usagé, ces conteneurs comprenant des moyens visant à interdire le retrait de l'objet une fois déposé ; de tels conteneurs comportent généralement à leur face supérieure une ouverture à bascule et sur leur fond une trappe à glissière pour pouvoir en effectuer la vidange.

On connaît par un brevet US-3 240 373 une fermeture de sécurité pour bouteille dans laquelle une trappe à coulisseau vient refermer, après encliquetage une ouverture ; le bord de l'ouverture n'est pas pourvu d'encoches et l'encliquetage n'est pas irréversible.

On connaît aussi par un brevet US-A-4 375 849 une boîte comportant au-dessus de son couvercle une pièce rotative pourvue d'une ouverture destinée à venir, par rotation de l'opercule, en regard d'une lumière pourvue de crans du couvercle ; les crans de la lumière agissent à la manière d'une clé pour permettre le dévissage de l'aiguille ; il n'est pas prévu de moyens de fermeture irréversible du couvercle.

On connaît enfin par un brevet US-A-3 792 803 une fermeture de flacon du genre de celle du premier brevet cité, dans laquelle le coulisseau est rappelé en position de fermeture par un élastique ; le bord de l'ouverture ne comporte pas d'encoches et il n'existe pas de moyens assurant l'irréversibilité de la fermeture.

Aucun des dispositifs précités n'est appliqué à l'extraction d'aiguilles emboîtées retirables par traction. Or les problèmes que se propose de résoudre l'invention sont les suivants : en premier lieu il s'agit de pouvoir fermer de façon irréversible la boîte, lorsque celle-ci a été remplie d'aiguilles, pour l'acheminer vers un incinérateur, par exemple ; en second lieu il s'agit de donner aux principaux éléments constitutifs de cette boîte, réceptacle et couvercle, une conformation leur permettant d'être transportés et stockés avec un minimum d'encombrement.

Plus précisément, le but de la présente invention est de proposer une structure de boîte de récupération utilisable dans les hôpitaux, cliniques, laboratoires, officines et d'une manière générale en tout lieu où l'on désire mettre commodément à l'écart de façon sûre de petits objets ou déchets tels qu'aiguilles d'injection, lames, tampons de soin, échantillons ou ampoules vides, etc...

Selon la présente invention, une boîte récupératrice de produits ou petits objets dangereux est remarquable notamment en ce qu'étant constituée d'une partie supérieure, dite couvercle, et d'une partie inférieure, dite réceptacle, le couvercle comporte sur sa face supérieure une ouverture obturable par une fermeture, et se présente sous une forme tronc-pyramidale ou analogue permettant d'emboîter plusieurs couvercles les dans les autres, tandis que le réceptacle a lui aussi une forme tronc-pyramidale permettant d'emboîter plusieurs réceptacles les uns dans les autres, le couvercle et le réceptacle étant pourvus le long de leur bord d'ouverture de moyens d'encliquetage irréversible interdisant leur séparation après qu'ils aient été assemblés.

De préférence, la fermeture du couvercle est une fermeture à glissière constituée d'un coulisseau et d'un couple de coulisse, le coulisseau étant constamment sollicité en position de fermeture au-dessus de l'ouverture par un moyen élastique.

De préférence, un bord de l'ouverture comporte trois encoches rectangulaires et/ou semi-circulaires destinées au désengagement des aiguilles d'injection ou de prélèvement de diverses sections ; avantageusement le couvercle comporte, de part et d'autre de la glissière un couple d'encoches destinées à la préhension digitale.

De préférence, le fond du réceptacle est bombé intérieurement pour former un logement épousant la partie supérieure du couvercle et conférer ainsi une certaine stabilité à un empilement de boites.

De préférence enfin, les parties constitutives essentielles, à savoir : le couvercle, le réceptacle et la fermeture, sont réalisées en matière plastique ; avantageusement, ladite matière plastique est un polypropylène.

La présente invention sera mieux comprise et des détails en relevant apparaîtront à la description qui va être faite d'une forme particulière de réalisation en relation avec les figures des planches annexées, dans lesquelles :

– la fig.1 est une illustration en perspective d'une boîte récupératrice de l'invention,

– la fig.2 illustre schématiquement les empilements des deux parties séparables de la boîte de la fig.1,

– la fig.3 illustre de façon analogue l'empilement de deux boîtes,

– la fig.4 est une coupe transversale du couvercle suivant AA,

– la fig.5 est une vue partielle de dessus de ce dernier, et la fig.5a est une coupe correspondante, et

– la fig.6 en est une vue analogue de dessous.

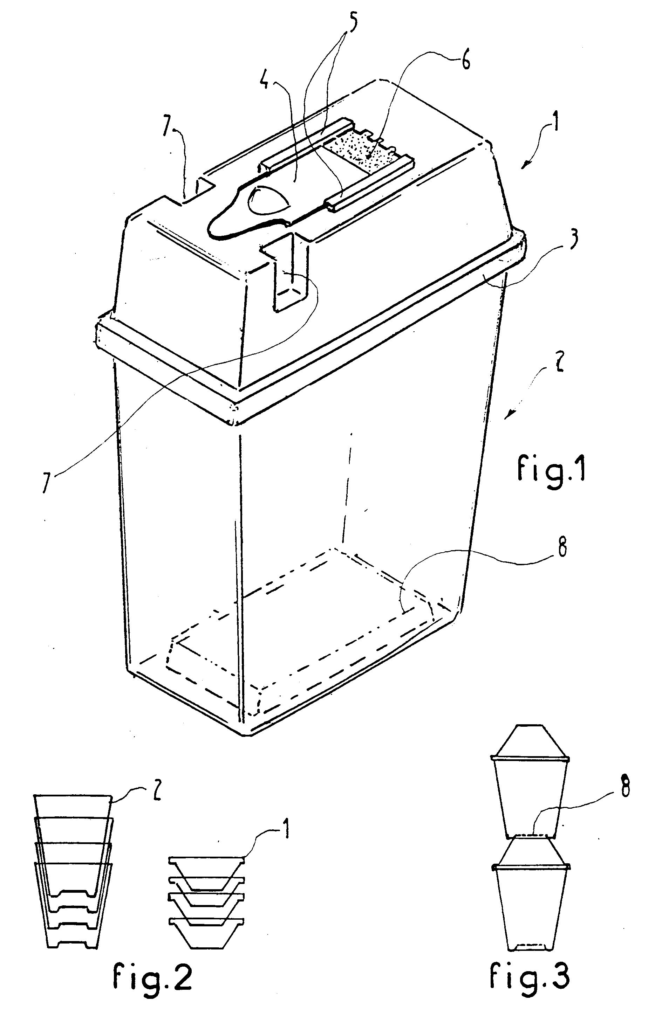

Sur la fig.1, une boîte récupératrice de l'invention se compose essentiellement d'un couvercle 1 et d'un réceptacle 2, le couvercle étant emboîté sur le réceptacle au moyen d'un rebord 3 dont la structure fonctionnelle sera décrite plus loin. Le couvercle aussi bien que le réceptacle sont de forme tronc-pyramidale mais ils pourraient tout aussi bien être de forme tronconique ou encore d'une forme géométrique intermédiaire entre ces deux formes.

On remarque sur la figure que le couvercle comporte sur sa face supérieure une fermeture à glissière constituée d'un coulisseau 4 pouvant glisser dans un couple de coulisses 5 pour obturer une ouverture 6 donnant accès à l'intérieur de la boîte ; on remarque en outre sur le couvercle un couple d'encoches 7 disposées de part et d'autre de la fermeture grâce auquel le couvercle peut être tenu fermement entre le pouce et. le doigt majeur tandis que l'index sollicite le coulisseau en ouverture. On indique que dans une autre forme de réalisation non représentée sur les figures, le coulisseau et ses coulisses pourraient être disposés sous la face interne du couvercle, seule n'apparaissant sur la face externe une manette passant à travers la face supérieure du couvercle.

On remarque en outre sur cette même figure que le fond du réceptacle est bombé intérieurement pour former un logement 8.

Sur la fig.2, il apparaît comment, aussi bien les réceptacles 2 que les couvercles 1 peuvent être emboîtés respectivement les uns dans les autres pour présenter, au conditionnement, un encombrement minimal.

Sur la fig.3, il apparaît comment grâce au logement 8 du fond d'une boîte, celle-ci peut être posée de façon stable au-dessus d'une boîte semblable ce qui permet de positionner convenablement les boîtes dans un emballage d'évacuation.

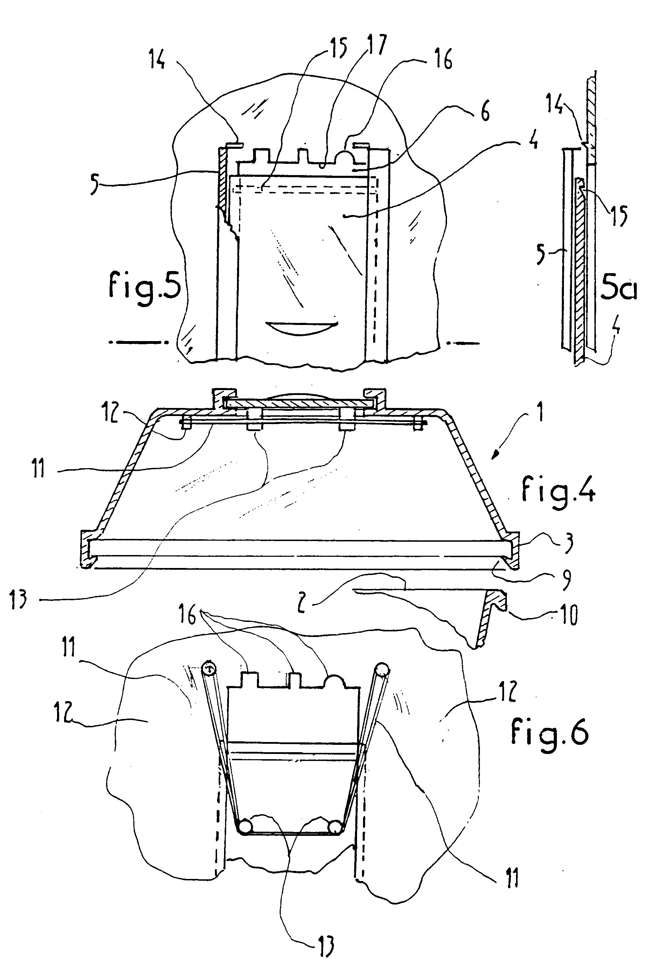

Sur la fig.4, on remarquera notamment la structure du bord 3 du couvercle 2 conformé intérieurement en nervure à bord 9 rabattu vers l'intérieur ; ce bord 9 est destiné lui-même à coopérer avec un rebord en saillie 10 du réceptacle 2 ; il découle de ces conformations des bords du réceptacle et du couvercle qu'un emboîtement, ou encliquetage, en force de l'un dans l'autre sera pratiquement irréversible et que la boite réceptrice, une fois fermée, ne pourra plus être réouverte par mégarde.

Sur les fig.4 et 6 apparaît un bracelet en caoutchouc 11 servant de moyen de sollicitation du coulisseau 4 vers sa position de fermeture ; ce bracelet est tendu entre deux ergots 12 du couvercle et deux ergots 13 du coulisseau. En lieu et place du bracelet, on pourrait concevoir que le moyen de sollicitation, ou de rappel, soit constitué par un fil d'acier, dit corde à piano, ou encore par un ou deux petits ressorts à spirale.

Sur la fig.5 plus particulièrement, il apparaît que la face supérieure du couvercle comporte au voisinage des extrémités des coulisses 5, un couple de crans 14 s'interposant sur le trajet du coulisseau 4 et formant une butée de fin de course pour celui-ci ; il apparaît aussi que l'extrémité du coulisseau comporte sur sa face inférieure une encoche 15. Lorsque, par une forte pression, on oblige le coulisseau à franchir les crans 14, ces derniers viendront en prise dans l'encoche 15 et interdiront l'ouverture par simple pression de l'index. La boîte réceptrice sera alors complètement fermée.

Sur les fig.5 et 6, on remarque enfin des encoches rectangulaires et/ou semi-circulaires telles que 16 disposées le long d'un bord transversal 17 de l'ouverture 6. Ces encoches qui peuvent être de dimensions différentes, sont destinées à permettre la prise de l'embout des aiguilles d'injection en vue de désengager celle-ci de la seringue, ou des corps du matériel d'injection, grâce à quoi l'utilisateur n'a pas à toucher l'aiguille elle-même pour la retirer de la seringue.

Bien que l'on ait décrit et représenté une forme particulière de réalisation de l'invention, il doit être compris que la portée de l'invention n'est pas limitée à cette forme, mais qu'elle s'étend à toute boîte récupératrice comportant les caractéristique générales énoncées plus haut.

**Revendications**

1.- Boîte récupératrice de produits ou petits objets dangereux tels qu'aiguilles d'injections, ladite boîte étant principalement constituée d'un réceptacle (2) et d'un couvercle (1), pouvant être emboîtés l'un sur l'autre par encliquetage irréversible (9,10), le couvercle comportant une ouverture obturable (6) du type trappe à glissière, constituée d'un coulisseau (4) et d'un couple de coulisses (5), ledit coulisseau comportant des moyens (14,15) d'interdiction d'ouverture par simple pression sur le coulisseau, caractérisée en ce que :

le bord transversal (17) de l'ouverture (6) est pourvu d'encoches (16) destinées à permettre la prise de l'embout des aiguilles d'injection,

le coulisseau (4) de la trappe à glissière est constamment sollicité en position de fermeture au-dessus de l'ouverture par un bracelet en caoutchouc (11) tendu entre au moins un ergot (12) du couvercle et un ergot (13) du coulisseau, et

le couvercle (1) comporte, de part et d'autre de la glissière (5) un couple d'encoches (7) destinées à la préhension digitale ;

2.- Boîte récupératrice selon la revendication 1, caractérisée en ce que :

le réceptacle (2) et le couvercle (1) sont pourvus de moyens (9,10) permettant leur emboîtement l'un sur l'autre par encliquetage irréversible,

la trappe comporte des moyens (14,15) d'interdiction d'ouverture par simple pression sur le coulisseau, consistant en un couple de crans (14) s'interposant sur le trajet du coulisseau (4) et formant une butée de fin de course pour acheminer, l'extrémité du coulisseau comportant sur sa face inférieure une encoche (15), disposée de telle sorte que les crans (14) puissent par forte pression sur le coulisseau venir en prise dans l'encoche (14) ;

3.- Boîte récupératrice selon la revendication 1, caractérisée en ce que:

le couvercle (1) et le réceptacle (2) sont d'une forme comprenant la forme tronc-pyramidale et la forme tronc-conique, leur permettant d'être emboîtés, respectivement, les uns dans les autres (fig.2), et

le fond du réceptacle est bombé intérieurement pour former un logement (8) épousant la partie supérieure du couvercle (1) et conférer ainsi une certaine stabilité à un empilement de boîte (fig.3).

## Patentansprüche

1. Abfallbehälter für gefährliche Produkte oder kleine Gegenstände wie Injektionsnadeln, im wesentlichen bestehend aus einem Gefäß (2) und einem Deckel, die irreversibel ineinander einrasten können (9, 10), wobei der Deckel eine verschließbare Öffnung (6) nach Art einer Schiebeklappe aufweist, bestehend aus einem Schieber (4) und einem Paar von Kulissen (5), wobei der Schieber Mittel (14, 15) zum Verhindern des Öffnens durch gewöhnlichen Druck auf den Schieber versehen ist, dadurch gekennzeichnet,

daß der Querrand (17) der Öffnung (6) mit Kerben (169) versehen ist, die zur Aufnahme der Ausbauchung der Injektionsnadeln dienen;

daß der Schieber (4) der Schiebeklappe über der Öffnung ständig in Schließposition gehalten ist durch ein Gummiband (11), das zwischen wenigstens einem Zapfen (12) des Deckels und einem Zapfen (13) des Schiebers gespannt ist, und

daß der Deckel (1) beidseits der Kulissen (5) ein Paar Senken (7) aufweist, die zum Erfassen mit den Fingern dienen.

2. Abfallbehälter nach Anspruch 1, dadurch gekennzeichnet,

daß das Gefäß (2) und der Deckel (1) mit Mitteln (9, 10) versehen sind, die deren gegenseitiges irreversibles Ineinanderschnappen erlauben,

daß die Klappe Mittel (14, 15) zum Verhindern des Öffnens durch gewöhnlichen Druck auf den Schieber umfaßt, bestehend aus einem Paar von Zacken (14), die sich in der Bewegungsbahn des Schiebers (4) befinden und einen Endanschlag bilden, um das Ende des Schiebers aufzunehmen, das auf seiner unteren Fläche eine Kerbe (15) aufweist, die derart angeordnet ist, daß die Zacken durch starken Druck auf den Schieber mit der Kerbe (15) in Eingriff gelangen.

3. Abfallbehälter nach Anspruch 1, dadurch gekennzeichnet,

daß der Deckel (1) und das Gefäß (2) eine Gestalt aufweisen, die einen Pyramidenstumpf und einen Kegelstumpf umfassen, so daß sie ineinander schachtelbar sind (Figur 2), und

daß der Boden des Gefäßes im Inneren eine Einbeulung (8) aufweist, die den oberen Teil des Deckels (1) aufnimmt und somit einem Stapel von Behältern eine gewisse Stabilität verleiht (Figur 3).

## Claims

1.- A gathering box for dangerous products or small objects such as injection needles, said box being primarily formed of a receptacle (2) and a lid (1) which can be fitted one on the other by irreversible latching (9,10), the cover having a closable opening (6) of the slide-trap type formed of a slide (4) and pair of slideways (5), said slide having means (14,15) to prevent opening by simple pressure on the slide, characterized by the fact that :

the transverse edge (17) of the opening (6) is provided with notches (16) intended to permit engagement with the tip of injection needles ;

the slide (4) of the slide-trap is constantly urged into closed position over the opening by a rubber band (11) stretched between at least one spur (12) on the lid and one spur (13) on the slide ; and

the lid (1) is provided, on both sides of the slide (5) with a pair of notches (7) intended for grasping by one's fingers ;

2.- A gathering box according to claim 1, characterized by the fact that :

the receptacle (2) and the lid (1) are provided with means (9,10) which permit their fitting one on the other by irreversible latching,

the trap has means ( 14, 15 ) for preventing opening by simple pressure on the slide, consisting of a pair of projections (14) present in the path of the slide (4) and forming a limit-stop for movement, the end of the slide being provided on its lower face with a notch (15) arranged in such a manner that the projections (14) can engage in the notch (15) by strong pressure on the slide ;

3.- A gathering box according to claim 1, characterized by the fact that :

the lid (1) and the cover (2) are of a shape comprising a frusto-pyramidal shape and a frusto-conical shape, enabling them to be fitted respectively one within the other (Fig.2) ; and

the bottom of the receptacle is curved inwardly to form a recess (8) which fits the upper part of the lid (1) and thus confers a certain stability on a stack of boxes (Fig.3).

fig.1

fig.2

fig.3

fig.5

fig.4

fig.6